(19)

Europäisches
Patentamt
European
Patent Office
Office européen
des brevets

(11) **EP 1 580 178 B1**

(12) **EUROPEAN PATENT SPECIFICATION**

(45) Date of publication and mention
of the grant of the patent:
**21.09.2011   Bulletin 2011/38**

(51) Int Cl.:
***C04B 35/119*** *(2006.01)*       ***C04B 35/488*** *(2006.01)*
***C04B 35/622*** *(2006.01)*

(21) Application number: **05006264.5**

(22) Date of filing: **22.03.2005**

(54) **ZrO2-Al2O3 composite ceramic material and production method thereof**

ZrO2-Al2O3 keramischer Verbundwerkstoff und Verfahren zu dessen Herstellung

ZrO2-Al2O3 matériau composite céramique et procédé de production de ce matériau

(84) Designated Contracting States:
**AT BE BG CH CY CZ DE DK EE ES FI FR GB GR
HU IE IS IT LI LT LU MC NL PL PT RO SE SI SK TR**

(30) Priority:  **23.03.2004   JP 2004085102**

(43) Date of publication of application:
**28.09.2005   Bulletin 2005/39**

(73) Proprietor: **Panasonic Electric Works Co., Ltd.**
**Kadoma-shi**
**Osaka (JP)**

(72) Inventors:
• **Nawa, Masahiro**
**Kadoma-shi,**
**Osaka 571-8686 (JP)**
• **Nakanishi, Hideo**
**Kadoma-shi,**
**Osaka 571-8686 (JP)**

• **Suehiro, Yasuhiko**
**Kadoma-shi,**
**Osaka 571-8686 (JP)**

(74) Representative: **Appelt, Christian W.**
**Forrester & Boehmert**
**Pettenkoferstrasse 20-22**
**80336 München (DE)**

(56) References cited:
**EP-A- 1 382 586     US-A- 5 863 850**

• **NAWA M ET AL: "The Effect of TiO2 Addition on
Strengthening and Toughening in Intragranular
Type of 12Ce-TZP/Al2O3 Nanocomposites"
JOURNAL OF THE EUROPEAN CERAMIC
SOCIETY, ELSEVIER SCIENCE PUBLISHERS,
BARKING, ESSEX, GB, vol. 18, no. 3, 1998, pages
209-219, XP004101497 ISSN: 0955-2219**

**Description**

TECHNICAL FIELD

The present invention relates to a $ZrO_2$-$Al_2O_3$ composite ceramic material with excellent mechanical properties, and a method of producing the same.

BACKGROUND ART

As compared with metal and plastic materials, ceramic materials demonstrate excellent hardness, wear resistance, heat resistance and corrosion resistance. As for practical use of the ceramic materials in various application fields, for example, biomaterial parts such as artificial joint and artificial tooth, medical equipments, cutting tools such as drill and surgical knife, mechanical parts for automobile, airplane and space craft used under severe conditions, it is desired to develop a ceramic material having further improved mechanical strength and toughness in a high level. In recent years, a zirconia ($ZrO_2$)-alumina ($Al_2O_3$) composite ceramic material has received attention as a potential candidate of the ceramic material.

For example, Japanese patent Early publication [kokai] No. 5-246760 discloses a $ZrO_2$-based composite ceramic sintered body comprising a matrix phase of tetragonal $ZrO_2$ grains containing 5 to 30 mol% of $CeO_2$ and a dispersion phase of fine grains of at least one of selected from the group consisting of $Al_2O_3$, $SiC$, $Si_3N_4$ and $B_4C$, which are dispersed within the $ZrO_2$ grains and at grain boundaries of the matrix phase. By the presence of the dispersion phase, a grain growth of the matrix phase is prevented and a fine grained structure of the matrix phase is obtained, so that significant strengthening is achieved mainly due to a reduction in flaw size of the fracture origin.

In addition, US patent No. 5,728,636 discloses a $ZrO_2$-based ceramic material having high mechanical strength and toughness, which comprises a tetragonal $ZrO_2$ phase of $ZrO_2$ grains having an average grain size of 5μm or less, and containing 8 to 12 mol% of $CeO_2$ and 0.05 to 4 mol% of $TiO_2$ as a stabilizer, and an $Al_2O_3$ phase of $Al_2O_3$ grains having an average grain size of 2 μm or less. In this ceramic material, the $Al_2O_3$ grains are dispersed within the $ZrO_2$ grains at a dispersion ratio of 2% or more, which is defined as a ratio of the number of the $Al_2O_3$ grains dispersed in the $ZrO_2$ grains relative to the number of the entire $Al_2O_3$ grains dispersed in the ceramic material. In addition, by using the combination of $CeO_2$ and $TiO_2$ as the stabilizer, the grain growth of the $ZrO_2$ grains can be enhanced moderately, so that parts of the $Al_2O_3$ grains are effectively dispersed within the $ZrO_2$ grains, and a critical stress of a stress induced transformation from the tetragonal $ZrO_2$ to monoclinic $ZrO_2$ is increased.

By the way, as one potential approach for improving wear resistance and hardness of this kind of the ceramic material, it is proposed to increase the additive amount of $Al_2O_3$. However, such an increase of the $Al_2O_3$ amount generally leads to reductions in mechanical strength and toughness. In above cases, a preferred amount of $Al_2O_3$ in the composite ceramic sintered body or the ceramic material is in a range of 0.5 to 50 vol%. When the $Al_2O_3$ amount exceeds 50 vol%, $Al_2O_3$ becomes the matrix phase, so that it is difficult to maintain a strengthening mechanism based on a stress induced phase transformation of $ZrO_2$. Consequently, considerable reductions in mechanical strength and toughness may occur.

Further, from Nawa M. et al, "The Effect of TiO2 Addition on Strengthening and Toughening in Intragranular Type of 12Ce-TZP/Al2O3 Nanocomposites", Journal of the European Ceramic Society, Barking, Essex, GB, vol. 18, No. 3, 1998, pages 209 to 219, a $ZrO_2$-$Al_2O_3$ composite material having an intragranular nanostructure is known in which submicron sized $Al_2O_3$ particles are dispersed within $ZrO_2$ grains and an intragranular microstructure in which ether $Al_2O_3$ particles or $ZrO_2$ particles are trapped within $ZrO_2$ or $Al_2O_3$ grains..

Furthermore, document EP 1 382 586 A2 discloses a $ZrO_2$-$Al_2O_3$ composite ceramic material including $Al_2O_3$ grains dispersed within $ZrO_2$ grains at a first dispersion ratio of 2% or more which is defined as a ratio of the number of the $Al_2O_3$ grains dispersed within the $ZrO_2$ grains relative to the number of the entire $Al_2O_3$ grains dispersed in the ceramic material and $ZrO_2$ grains at a second dispersion ratio of 1% or more which is defined as a ratio of the number of the $ZrO_2$ grains dispersed within the $Al_2O_3$ grains relative to the number of the entire $ZrO_2$ grains dispersed in the ceramic material.

Additionally, from document US 5 863 850 A a $ZrO_2$-$Al_2O_3$ composite ceramic material is known, where fine $Al_2O_3$ grains having an average grain size of 1 μm or less are dispersed within the grains of the partially stabilized zirconia at a dispersion ratio. The dispersion ratio is defined as a ratio of the number of $Al_2O_3$ grains dispersed within the grains of partially stabilized zirconia relative to the number of the entire $Al_2O_3$ grains dispersed in the ceramic material.

However, the previous $ZrO_2$-$Al_2O_3$ composite ceramic materials still have a problem to be solved for providing excellent wear resistance and hardness without causing reductions in mechanical strength and toughness under a larger amount of $Al_2O_3$.

## SUMMARY OF THE INVENTION

Therefore, a primary concern of the present invention is to provide a $ZrO_2$-$Al_2O_3$ composite ceramic material having increased wear resistance and hardness, while maintaining a good balance between mechanical strength and toughness, under a larger amount of $Al_2O_3$ than heretofore.

That is, the $ZrO_2$-$Al_2O_3$. composite ceramic material of the present invention comprises a $ZrO_2$ phase composed of 90 vol% or more of tetragonal $ZrO_2$, and an $Al_2O_3$ phase, wherein an amount of said $Al_2O_3$ phase in the composite ceramic material is in a range of 20 to 70 vol%, and the composite ceramic material comprises composite grains dispersed therein, each of which has a structure that an $Al_2O_3$ grain containing a fine $ZrO_2$ grain therein is trapped within a $ZrO_2$ grain, wherein a ratio of the number of the $Al_2O_3$ grains each existing in said composite particle and having the fine $ZrO_2$ grain therein relative to the number of the entire $Al_2O_3$ grains dispersed in the composite ceramic material is 0,3 % or more.

In the above composite ceramic material, it is preferred that the $ZrO_2$ phase contains 10 to 12 mol% of $CeO_2$ as a stabilizer.

In addition, it is preferred that a first dispersion ratio of the number of $Al_2O_3$ grains dispersed in $ZrO_2$ grains relative to the number of the entire $Al_2O_3$ grains dispersed in the composite ceramic material is 1,5% or more. Specifically, the first dispersion ratio defines a ratio of a total of the number of $Al_2O_3$ grains of the composite particles, each of which has the fine $ZrO_2$ grain therein and is trapped within the $ZrO_2$ grain, and the number of $Al_2O_3$ grains, each of which does not have the fine $ZrO_2$ grain therein and is trapped within the $ZrO_2$ grain, relative to the entire $Al_2O_3$ grains dispersed in the composite ceramic material. When the first dispersion ratio is 1,5 % or more, the composite ceramic material can be more effectively reinforced by the $Al_2O_3$ grains dispersed within the $ZrO_2$ grains. Consequently, the mechanical properties of the composite ceramic material of the present invention can be further improved.

In addition, it is preferred that a second dispersion ratio of the number of $ZrO_2$ grains disperused in $Al_2O_3$ grains relative to the number of the entire $ZrO_2$ grains dispersed in the composite ceramic material is 4% or more. Specifically, the second dispersion ratio defines a ratio of a total of the number of fine $ZrO_2$ grains, which are trapped within the $Al_2O_3$ grains constructing the composite particles, and the number of $ZrO_2$ grains, which are trapped within the $Al_2O_3$ grains not constructing the composite particles, relative to the number of the entire $ZrO_2$ grains dispersed in the composite ceramic material. When the second dispersion ratio is 4% or more, it is possible to increase an amount of zirconia toughened alumina (ZTA) formed by the fine tetragonal $ZrO_2$ grains trapped within the $Al_2O_3$ grains, as described later. Consequently, the composite ceramic material of the present invention demonstrates mechanical properties with a higher degree of reliability.

In the present invention, the improvement in mechanical properties of the composite ceramic material has been achieved by aggressively dispersing the composite particles in the composite ceramic material to consequently increase the formation amount of zirconia toughened alumina (ZTA) therein.

A further concern of the present invention is to provide a method of producing the $ZrO_2$-$Al_2O_3$ composite ceramic material described above. That is, this method comprises the steps of:

mixing a first powder for providing said $ZrO_2$ phase with a second powder for providing said $Al_2O_3$ phase such that an amount of said $Al_2O_3$ phase in the composite ceramic material is in a range of 20 to 70 vol%, the second powder containing $Al_2O_3$ particles each having a fine $ZrO_2$ particle therein;

molding a resultant mixture in a desired shape to obtain a green compact; and sintering said green compact in an oxygen-containing atmosphere, so that the composite ceramic material comprises composite particles dispersed therein, each of which has a structure that an $Al_2O_3$ grain containing a fine $ZrO_2$ gain therein is trapped within a $ZrO_2$ grain, and a ratio of the number of the $Al_2O_3$ grains existing in said composite particle and having the fine $ZrO_2$ grain therein relative to the number of the entire $Al_2O_3$ grains dispersed in the composite ceramic material is 0,3% or more.

As a preferred preparation process of the second powder in the above method, it comprises the step of adding a $ZrO_2$ powder to at least one selected from a θ-$Al_2O_3$ powder and a γ-$Al_2O_3$ powder having a specific surface area of 50 to 400 $m^2$/g to obtain a mixed powder, and then calcining the mixed powder in an oxygen atmosphere at a temperature of 800°C to 1300°C. In addition, it is preferred that the preparation process comprises the steps of adding a $ZrO_2$ powder to one of an aqueous solution of an aluminum salt and an organic solution of an aluminum alkoxide, hydrolyzing a resultant mixture to obtain a precipitate, and drying the precipitate, and then calcining the precipitate in an oxygen containing atmosphere at a temperature of from 800°C to 1300°C. Alternatively, it is preferred that the preparation process comprises the steps of adding an aqueous solution of a zirconium salt to one of an aqueous solution of an aluminum salt and an organic solution of an aluminum alkoxide, hydrolyzing a resultant mixture to obtain a precipitate, and drying the precipitate, and then calcining the precipitate in an oxygen containing atmosphere at a temperature of from 800°C to 1300°C.

From the viewpoint of more efficiently dispersing the composite particles in the composite ceramic material of the present invention, it is particularly preferred that the second powder is mainly composed of $\alpha$-$Al_2O_3$ particles having an average particle size of 0,3 $\mu$m or less, each of which contains a fine $ZrO_2$ particle therein. In this case, it is possible to promote the formation of the composite particles during the sintering step, and consequently increase the amount of zirconia toughened (ZTA) in the composite ceramic material.

Without wishing to be bound by theory, it is presently believed that the remarkable improvement of mechanical properties in the present invention results from the following mechanism. As described above, the ZrO2-Al2O3 composite ceramic material of the present invention is characterized by the composite particles dispersed therein, each of which has the structure that the Al2O3 grain having the fine (tetragonal) ZrO2 grain therein is trapped within the (larger) ZrO2 grain. Since the fine (tetragonal)

$ZrO_2$ grain trapped within the $Al_2O_3$ grain provides the zirconia toughened alumina (ZTA), the toughness of the $Al_2O_3$ grain is remarkably improved by the presence of the fine $ZrO_2$ grain. When such a toughness-improved $Al_2O_3$ grain is trapped within the (larger) $ZrO_2$ grain, sub-grain boundaries are formed within the $ZrO_2$ grain. The formation of the sub-grain boundaries plays a role in dividing the (larger) $ZrO_2$ grain, incorporating the toughness-improved $Al_2O_3$ grain therein, into imaginary more finer sized grains.

In addition, a residual stress field generated within the (larger) $ZrO_2$ grain increases a critical stress required for causing a stress induced phase transformation from tetragonal $ZrO_2$ to monoclinic $ZrO_2$. Furthermore, in the present invention, the dispersion of the composite particles (In the present specification, the structure of the composite particle is named as "a triple nanocomposite structure".) remarkably reduces the average grain size of $ZrO_2$ and $Al_2O_3$ grains constructing the composite ceramic material. Thus, according to such a unique structural control at nanometer levels, it is possible to provide the $ZrO_2$-$Al_2O_3$ composite ceramic material having excellent wear resistance and hardness, while maintaining the good balance between mechanical strength and toughness, under a larger amount, i.e., 40 to 70 vol% of $Al_2O_3$ in the composite ceramic material.

These and further purposes and advantages of the present invention will be clearly understood from the following detail explanation of the invention.

BRIEF EXPLANATION OF THE DRAWING

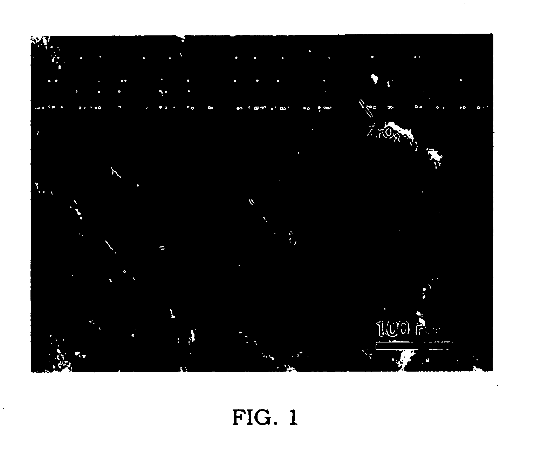

FIG. 1 is a SEM photograph showing a composite particle dispersed in a $ZrO_2$-$Al_2O_3$ composite ceramic material of the present invention.

DETAIL EXPLANATION OF THE INVENTION

The $ZrO_2$ phase of the $ZrO_2$-$Al_2O_3$ composite ceramic material of the present invention is composed of 90 vol% or more of tetragonal $ZrO_2$. To obtain such a large amount of tetragonal $ZrO_2$, it is preferred that the $ZrO_2$ phase contains 10 to 12 mol% of $CeO_2$ as a stabilizer. When the $CeO_2$ amount is less than 10 mol%, an amount of monoclinic $ZrO_2$ relatively increases, so that cracks may easily occurs in the composite ceramic material. On the other hand, when the $CeO_2$ amount exceeds 12 mol%, cubic $ZrO_2$ of a high-temperature stable phase begins to appear, so that there is a fear that the mechanical strength and toughness can not be sufficiently improved by the stress induced phase trans-formation of tetragonal $ZrO_2$ to monoclinic $ZrO_2$. Preferably, the zirconia phase is composed of 90 vol% or more of tetragonal $ZrO_2$ and the balance of monoclinic $ZrO_2$.

The composite ceramic material of the present invention is essential to contain 20 to 70 vol%., and preferably 40 to 60 vol% of the $Al_2O_3$ phase. When the $Al_2O_3$ amount is less than 20 vol%, the wear resistance and the mechanical strength of the composite ceramic material can not be sufficiently improved. On the other hand, when the $Al_2O_3$ amount exceeds 70 vol%, considerable reductions in mechanical strength and toughness occur. When the $Al_2O_3$ amount is in the range of 40 to 60 vol%, it is possible to provide a high-reliability ceramic material having good balance between the mechanical strength and toughness in a high level.

The most important feature of the composite ceramic material of the present invention is to aggressively disperse the composite particles to the composite ceramic material, each of which has the structure that the $Al_2O_3$ grain containing a fine (tetragonal) $ZrO_2$ grain therein is trapped within a $ZrO_2$ grain, as shown in FIG. 1.

In the present invention, the ratio of the number of the $Al_2O_3$ grains each existing in the composite particle and having the fine $ZrO_2$ grain therein relative to the number of the entire $Al_2O_3$ grains dispersed in the composite ceramic material is 0.3 % or more. When this ratio is less than 0.3 %, the formation amount of the zirconia toughened alumina (ZTA) in the composite ceramic material decreases, so that there is a fear that the effect of improving the mechanical strength and toughness is not sufficiently obtained as increasing $Al_2O_3$ content. In other words, as this ratio is larger than 0.3 %, a much higher improvements of both mechanical strength and toughness of the composite ceramic material can be obtained.

It is also preferred that a first dispersion ratio of the number of $Al_2O_3$ grains dispersed in the $ZrO_2$ grains relative to the number of the entire $Al_2O_3$ grains dispersed in the composite ceramic material is 1.5 % or more. When the first dispersion ratio is less than 1.5 %, an effect of dividing the $ZrO_2$ grains into more finer sized grains by the formation of sub-grain boundaries may become insufficient, so that a reduction in strength easily occurs as increasing $Al_2O_3$ content. An upper limit of the first dispersion ratio is not restricted. In a theoretical sense, as the first dispersion ratio increases, the mechanical properties of the composite ceramic material can be further improved. The number of $Al_2O_3$ grains each existing in the composite particle is included in the number of the $Al_2O_3$ grains dispersed in the $ZrO_2$ grains.

It is preferred that a second dispersion ratio of the number of $ZrO_2$ grains dispersed in the $Al_2O_3$ grains relative to the number of the entire $ZrO_2$ grains dispersed in the composite ceramic material is 4 % or more. When the second dispersion ratio is less than 4 %, the formation amount of the zirconia toughened alumina (ZTA) decreases, so that the effect of improving the mechanical properties of the composite ceramic material may become insufficient. In particular, when the second dispersion ratio is less than 4 % in the presence of a large amount of $Al_2O_3$ content, a reduction in strength easily occurs. An upper limit of the second dispersion ratio is not restricted. In a theoretical sense, as the second dispersion ratio increases, the mechanical properties of the composite ceramic material can be further improved.

The size of the fine $ZrO_2$ grain of the composite particle is not restricted on the assumption that the fine $ZrO_2$ grain can be trapped within the $Al_2O_3$ grain. For example, it is preferred that fine tetragonal $ZrO_2$ grains having an average grain size of several ten nanometers are trapped within the $Al_2O_3$ grains. The number of the fine $ZrO_2$ grains each trapped within the $Al_2O_3$ grain of the composite particle is included in the number of the $ZrO_2$ grains dispersed in the $Al_2O_3$ grains.

It is preferred that the $Al_2O_3$ grains of the composite ceramic material has an average grain size of 0.1 to 0.5 $\mu$m. When the average grain size exceeds 0.5 $\mu$m, it becomes difficult to disperse the $Al_2O_3$ grains within the $ZrO_2$ grains at the above first dispersion ratio. On the other hand, when the average grain size is less than 0.1 $\mu$m, it is difficult to obtain a highly dense sintered body of the composite ceramic material by pressureless sintering.

The size of the $ZrO_2$ grain of the composite particle is determined such that the $Al_2O_3$ grain having the fine $ZrO_2$ grain therein is trapped within the $ZrO_2$ grain. However, when the size of the $ZrO_2$ grain is excessively large, it may lead to a reduction in strength of the composite ceramic material. From this viewpoint, it is preferred that an average grain size of the $ZrO_2$ grains of the composite ceramic material is in a range of 0.1 to 1 $\mu$m. This average grain size is based on the $ZrO_2$ grains other than the fine $ZrO_2$ grains trapped within the $Al_2O_3$ grains. When the average grain size exceeds 1 $\mu$m, reductions in wear resistance and mechanical strength may occur. On the other hand, when the average grain size is less than 0.1 $\mu$m, it becomes difficult to obtain a highly dense sintered body of the composite ceramic material by pressureless sintering.

By the way, in the case of a conventional composite ceramic material with a simply mixed structure of $ZrO_2$ and $Al_2O_3$ grains having an average grain size of several micron levels, when the $Al_2O_3$ amount exceeds 30 vol%, the toughening mechanism based on the stress induced phase transformation of tetragonal $ZrO_2$ to monoclinic $ZrO_2$ is not a dominant factor of the composite ceramic material, so that there is a tendency that the mechanical strength and toughness gradually decrease. In addition, when the $Al_2O_3$ amount exceeds 50 vol%, it means that the matrix phase of the composite ceramic material is provided by the $Al_2O_3$ phase. This leads to a considerable deterioration of the mechanical properties of the conventional composite ceramic material.

According to the $ZrO_2$-$Al_2O_3$ composite ceramic material of the present invention, in which the composite particles having the triple nanocomposite structure are dispersed, the fine $ZrO_2$ grains each trapped within the $Al_2O_3$ grain and the $Al_2O_3$ grains each trapped within the $ZrO_2$ grain contribute to promote piling up dislocations and form the sub-grain boundaries within the crystal grains, so that the wear resistance and mechanical strength of the composite ceramic material can be remarkably improved. In particular, when the $Al_2O_3$ amount is in the range of 40 to 60 vol%, fine tetragonal $ZrO_2$ grains are uniformly dispersed in the $Al_2O_3$ grains to form the zirconia toughened alumina (ZTA) structure, so that the $Al_2O_3$ grains are remarkably reinforced. In other words, even when the $Al_2O_3$ amount exceeds 50 vol%, high mechanical strength and toughness can be maintained by the formation of a fine crystal-grain structure effectively reinforced by the tetragonal $ZrO_2$ grains. From these reasons, the $ZrO_2$-$Al_2O_3$ composite ceramic material of the present invention obtained under the $Al_2O_3$ content larger than 50 vol% where the matrix phase is the $Al_2O_3$ phase exhibits excellent mechanical strength and toughness substantially equal to the former $ZrO_2$-$Al_2O_3$ ceramic material where the matrix phase is the $ZrO_2$ phase.

Without wishing to be bound by theory, it is presently believed that the mechanical properties of the composite ceramic material of the present invention are improved by the following mechanism. That is, when the composite particles are dispersed in the composite ceramic material, each of which has the structure that the $Al_2O_3$ grain containing the fine tetragonal $ZrO_2$ grain therein is trapped within the tetragonal $ZrO_2$ grain, a residual stress field is locally generated around each of the fine tetragonal $ZrO_2$ grains within the $Al_2O_3$ grains and around each of the $Al_2O_3$ grains within the tetragonal $ZrO_2$ grains by a difference in thermal expansion coefficient between $Al_2O_3$ and $ZrO_2$ during a cooling procedure after sintering. By the influence of the residual stress field, lots of dislocations easily occur within the respective crystal grains. The dislocations are then piled up with each other, and finally the sub-grain boundaries are formed within the $Al_2O_3$ and $ZrO_2$ grains, respectively. The sub-grain boundaries provide the finer-grained structure, which has the capability of increasing a critical stress required for causing the stress-induced phase transformation from the tetragonal $ZrO_2$ to the

monoclinic $ZrO_2$. As a result, the composite ceramic material of the present invention demonstrates excellent wear resistance and hardness as well as high mechanical strength and toughness.

Referring to the SEM photograph of FIG. 1, the structure of the composite ceramic material of the present invention is more concretely explained. This SEM photograph shows that the above-described composite particle exists in a uniformly mixed structure of normal tetragonal $ZrO_2$ grains not having $Al_2O_3$ grains therein, and normal $\alpha$-$Al_2O_3$ grains not having $ZrO_2$ grains therein. In addition, it shows that an $Al_2O_3$ grain containing a fine $ZrO_2$ grain therein and $Al_2O_3$ grains not containing the fine $ZrO_2$ grain therein are dispersed within the large $ZrO_2$ grain constructing this composite particle. Moreover, it shows that an $Al_2O_3$ grain containing a fine $ZrO_2$ grain therein other than the composite particle exists in the composite ceramic material. The number of fine $ZrO_2$ grains in the single $Al_2O_3$ grain and the number of $Al_2O_3$ grains in the single $ZrO_2$ grain are not restricted. For example, a plurality of fine $ZrO_2$ grains may be trapped in the single $Al_2O_3$ grain, or a plurality of $Al_2O_3$ grains may be trapped in the single $ZrO_2$ grain.

As a preferred embodiment of the present invention, the zirconia phase may contain another stabilizer such as MgO, CaO, $TiO_2$ and/or $Y_2O_3$ in addition to $CeO_2$. For example, it is preferred to use 0.01 to 1 mol% of $TiO_2$ and/or 0.01 to 0.5 mol% of CaO with respect to the total amount of the zirconia phase in addition to 10 to 12 mol% of $CeO_2$. In this case, the grain growth of the zirconia phase is enhanced to a moderate degree by the addition of $TiO_2$, so that $Al_2O_3$ grains can be easily dispersed within the $ZrO_2$ grains. In addition, it is possible to increase a critical stress of the stress induced phase transformation. When the additive amount of $TiO_2$ is less than 0.01 mol%, the effect of enhancing the grain growth of the zirconia phase may be not obtained sufficiently. On the other hand, when the additive amount of $TiO_2$ exceeds 1 mol%, abnormal grain growth of the zirconia phase easily occurs, so that the mechanical strength and the wear resistance of the composite ceramic material may deteriorate.

On the other hand, the addition of CaO prevents the abnormal grain growth of the zirconia phase to improve the balance between the mechanical strength and toughness. In particular, it is effective to obtain the composite ceramic material having excellent wear resistance and mechanical strength. When the additive amount of CaO is less than 0.01 mol%, the effect of preventing the abnormal grain growth of the zirconia phase may be not obtained sufficiently. On the other hand, when the additive amount of CaO exceeds 0.5 mol%, cubic zirconia beings to appear in the zirconia phase, so that it becomes difficult to obtain the zirconia phase composed of 90 vol% or more of tetragonal $ZrO_2$. The generation of cubic zirconia easily leads to an increase in average grain size of the zirconia phase. In such a case, deterioration in mechanical strength, toughness and wear resistance may occur. The zirconia phase may contain a small amount of impurities. For example, it is desired that the amount of the impurities is 0.5 mol% or less with respect to the total amount of the zirconia phase.

The composite ceramic material of the present invention is preferably used in applications requiring excellent wear resistance expected by increasing the $Al_2O_3$ content, while maintaining mechanical strength and toughness substantially equal to the previous $ZrO_2$-$Al_2O_3$ ceramic materials. For example, it is preferred to use the composite ceramic material of the present invention for an artificial joint described in the international patent application WO02/ 11780. That is, when a joint portion of the artificial joint is provided by a sliding contact between the composite ceramic material and polyethylene, it is possible to reduce a wear amount of polyethylene. In addition, when the joint portion of the artificial joint is formed by a sliding contact between the composite ceramic materials, particularly improved wear resistance can be achieved. Thus, by use of the composite ceramic material of the present invention, it is possible to obtain the artificial joint having the capability of stably providing a smooth joint motion for an extended time period under severe conditions in a living body.

Next, a method of producing the $ZrO_2$-$Al_2O_3$ composite ceramic material of the present invention is explained. The present method comprises the steps of mixing a first power for providing the $ZrO_2$ phase with a second powder for providing the $Al_2O_3$ phase such that an amount of the $Al_2O_3$ phase in the composite ceramic material is in a range of 20 to 70 vol%, molding a resultant mixture in a desired shape to obtain a green compact, and sintering the green compact at a sintering temperature in an oxygen-containing atmosphere, so that the composite ceramic material comprises composite particles dispersed therein, each of which has a structure that an $Al_2O_3$ grain containing a fine $ZrO_2$ grain therein is trapped within a $ZrO_2$ grain.

To obtain the $ZrO_2$ phase composed of 90 vol% or more of tetragonal $ZrO_2$, it is preferred that the first powder is prepared such that the $ZrO_2$ phase contains 10 to 12 mol% of $CeO_2$ as a stabilizer. In addition, as the first powder, it is preferred to use a tetragonal $ZrO_2$ powder containing a required amount of $TiO_2$ and/or CaO in addition to $CeO_2$. A preparation process of the first powder is not restricted. For example, the following process is recommended.

That is, a cerium containing compound such as cerium salts is added to an aqueous solution of a zirconium salt. If necessary, an aqueous solution of a titanium salt and/or a calcium salt, or an organic solution of a titanium or calcium alkoxide as a titanium containing compound or a calcium containing compound may be added. Then, hydrolysis is performed by adding an alkali aqueous solution such as aqueous ammonia to a resultant mixture to obtain a precipitate. The precipitate is dried, calcined in the oxygen-containing atmosphere, e.g., in the air, and then pulverized by means of wet ball milling to obtain the tetragonal $ZrO_2$ powder having a desired particle distribution.

In the case of using the tetragonal $ZrO_2$ powder, it is preferred that the $ZrO_2$ powder has a specific surface area of 10 to 20 m$^2$/g to obtain the green compact of a sufficient green density. Such a green compact can be easily sintered by pressureless sintering. When the specific surface area is less than 10 m$^2$/g, it becomes difficult to obtain the $ZrO_2$ phase having an average grain size of 1 $\mu$m or less after sintering. On the other hand, when the specific surface area exceeds 20 m$^2$/g, the bulk density considerably decreases, so that handling of the first powder becomes difficult. As a result, there is a fear that the green compact can not be densely sintered by pressureless sintering.

In the present invention, to uniformly disperse the composite particles consisting of the triple nanocomposite structure in the composite ceramic material, a composite powder comprising $Al_2O_3$ particles each containing a fine $ZrO_2$ particle therein is used as the second powder. For example, a required amount of the first powder is mixed with an $Al_2O_3$ powder to obtain a mixed powder, and then a resultant mixed powder is calcined in an oxygen containing atmosphere at a temperature of 800°C to 1300 °C, and preferably 1000 °C to 1200 °C to obtain the composite powder. In this case, it is preferred that the $Al_2O_3$ powder is at least one selected from a θ-$Al_2O_3$ powder and a γ-$Al_2O_3$ powder having a specific surface area of 50 to 400 m$^2$/g. The specific surface area of this $Al_2O_3$ powder is much greater than the specific surface of the first powder. In other words, since the $Al_2O_3$ powder used to prepare the composite powder is much finer than the first powder, the above-described mixed powder comprises $ZrO_2$ particles surrounded with ultra-fine $Al_2O_3$ particles.

Next, a phase transformation of θ-$Al_2O_3$ and/or γ-$Al_2O_3$ of the mixed powder to α-$Al_2O_3$ occurs during the calcining procedure. At this time, the $ZrO_2$ particles in the mixed powder are trapped within α-$Al_2O_3$ particles each having an increased particle size caused by the phase transformation. The thus obtained composite powder is excellent in moldability as compared with the case of using the θ-$Al_2O_3$ or γ-$Al_2O_3$ powder. In addition, there is an advantage that the average grain size of the $Al_2O_3$ particle dispersed in the composite ceramic material can be easily controlled in the range of 0.1 to 0.5 $\mu$m.

It is preferred that the composite powder obtained by the above preparation process is mainly composed of α-$Al_2O_3$ particles having an average grain size of 0.3 $\mu$m or less, each of which has the fine $ZrO_2$ particle therein. However, an amount of α-$Al_2O_3$ in the composite powder is not restricted. That is, it is enough that a part of θ-$A_2O_3$ and/or γ-$Al_2O_3$ is transformed to α-$Al_2O_3$ by the calcining procedure, and allowed to be mixed condition of θ-$Al_2O_3$ and/or γ-$Al_2O_3$ and α-$Al_2O_3$.

The preparation process of the second powder is not restricted. For example, a $ZrO_2$ powder is added to an aqueous solution of an aluminum salt or an organic solution of an aluminum alkoxide. A resultant mixture is hydrolyzed to obtain a precipitate, and then the precipitate is dried. The dried precipitate is calcined in an oxygen containing atmosphere at a temperature of from 800 °C to 1300 °C, and then pulverized by means of wet ball milling to obtain the second powder having a desired particle distribution. In the above method, an aqueous solution of a zirconium salt may be used in stead of the $ZrO_2$ powder.

In preparing the composite powder as the second powder, a mixing ratio of $Al_2O_3$ and $ZrO_2$ is not restricted. To efficiently obtain the α-$Al_2O_3$ particles each containing a fine $ZrO_2$ particle therein, it is preferred that a volume ratio of $Al_2O_3$ : $ZrO_2$ in the composite powder is in a range of 95 : 5 to 50 : 50. When the value of $ZrO_2$ in this volume ratio is less than 5, it is difficult to obtain sufficient amounts of the α-$Al_2O_3$ particles each containing a fine $ZrO_2$ particle therein by the calcining procedure. Consequently, the formation amount of the composite particles in the composite ceramic material decreases. On the other hand, when the value of $ZrO_2$ in this volume ratio is more than 50, an agglomeration of the $ZrO_2$ particles may occur. When the above volume ratio is within the range of 90 : 10 to 60 : 40, it is possible to more efficiently obtain the α-$Al_2O_3$ particles each containing a fine $ZrO_2$ particle therein, thereby providing a high-quality composite powder suitable for producing the composite ceramic material of the present invention.

If necessary, a HIP treatment may be performed in an oxygen-containing atmosphere after sintering. To obtain effects of the HIP treatment at the maximum, it is preferred that the sintered body of the composite ceramic material obtained by the pressureless sintering has a relative density of 95 % or more. A concentration of oxygen in the oxygen-containing atmosphere is not restricted. A mixture gas of oxygen and an inert gas such as argon may be used. In this case, it is preferred that the concentration of oxygen is approximately 5 vol% or more with respect to a total volume of the mixture gas.

EXAMPLES

The present invention is explained below according to preferred examples. The present invention is not limited to these Examples.

(Examples 1 to 6 and Comparative Examples 1 to 3)

A $ZrO_2$-$Al_2O_3$ composite ceramic material of each of Examples 1 to 6 and Comparative Examples 1 to 3 was produced by the following method. That is, as the first component for providing a $ZrO_2$ phase of the composite ceramic material, a tetragonal $ZrO_2$ powder having a specific surface area of 15 m$^2$/g and containing 11 mol% of $CeO_2$, 0.05

mol% of $TiO_2$, and 0.16 mol% of CaO was used. On the other hand, as a second component for providing an $Al_2O_3$ phase of the composite ceramic material, a composite powder comprised of a $\gamma$-$Al_2O_3$ powder having a specific surface area of 300 $m^2$/g and a part of the tetragonal $ZrO_2$ powder was used. A mixture ratio by volume of the $\gamma$-$Al_2O_3$ powder and the tetragonal $ZrO_2$ powder is 70 : 30.

The composite powder was prepared by the following procedures. That is, required amounts of the $\gamma$-$Al_2O_3$ powder and the tetragonal $ZrO_2$ powder were ball-milled in an ethanol solvent for 24 hours, and then dried to obtain a mixed powder. Subsequently, the mixed powder was calcined at 1000 °C in the air for 2 hours. The thus obtained calcined powder was further ball-milled in an ethanol solvent for 24 hours, and then dried to obtain the composite powder.

The remaining tetragonal $ZrO_2$ powder was mixed with the composite powder such that an $Al_2O_3$ amount in the composite ceramic material is in a range of 10 to 80 vol%, as listed in Table 1. A resultant mixture was ball-milled in an ethanol solvent for 24 hours, and then dried to obtain a powder for sintering. In Comparative Example 1, the $Al_2O_3$ content is zero.

The obtained powder for sintering was molded at the pressure of 10 MPa by uniaxial pressing to obtain a disk-shaped green compact having a diameter of about 68 mm. After a CIP (cold isostatic pressing) treatment was performed to the green compact at the pressure of 147 MPa, the green compact was sintered at the sintering temperature of 1440 °C for 3 hours in the air by pressureless sintering to obtain a sintering body.

With respect to each of Examples 1 to 6 and Comparative Examples 1 to 3, the sintered body has a relative density of more than 99%. By X-ray diffraction analysis, it was confirmed that the $ZrO_2$ phase of the respective sintered body is composed of 90 vol% or more of tetragonal $ZrO_2$ and the balance of monoclinic $ZrO_2$. In addition, by SEM (scanning electron microscope) and TEM (transmission electron microscope) observations, it was confirmed that the sintered body of each of Examples 1 to 6 and Comparative Examples 2 and 3 comprises composite particles dispersed therein, each of which has a triple nanocomposite structure that an $Al_2O_3$ grain containing a fine $ZrO_2$ grain therein is trapped within a $ZrO_2$ grain.

In addition, a first dispersion ratio (W1), which is defined as a ratio of the number of the $Al_2O_3$ grains dispersed within the $ZrO_2$ grains relative to the number of the entire $Al_2O_3$ grains dispersed in the composite ceramic material, second dispersion ratio (W2), which is defined as a ratio of the number of the $ZrO_2$ grains dispersed within the $Al_2O_3$ grains relative to the number of the entire $ZrO_2$ grains dispersed in the composite ceramic material, and a third dispersion ratio (W3), which is defined as a ratio of the number of $Al_2O_3$ grains each existing in the composite particle and containing the fine $ZrO_2$ grain therein relative to the number of the entire $Al_2O_3$ grains dispersed in the composite ceramic material, were listed in Table 2.

The first to third dispersion ratios (W1, W2, W3) were determined by the following method. First, a sample for observation was prepared by polishing the sintered body and performing a heat treatment to the polished surface. Then, the SEM observation of the sample or the TEM observation of the sintered body was performed to count the number (S1) of entire $Al_2O_3$ grains existing within a view field, the number (S2) of the entire $ZrO_2$ grains existing within the same view field, the number (n1) of $Al_2O_3$ grains dispersed within the $ZrO_2$ grains in the same view field, the number (n2) of the $ZrO_2$ grains dispersed within the $Al_2O_3$ grains in the same view field, and the number (n3) of $Al_2O_3$ grains, each of which exists in the composite particle and contains the fine $ZrO_2$ grain therein, in the same view field. By substituting these values to the following equations, those dispersion ratios were calculated. Results are shown in Tables 2.

$$W1\ [\%] = (n1\ /\ S1) \times 100,$$

$$W2\ [\%] = (n2\ /\ S2) \times 100.$$

$$W3\ [\%] = (n3\ /\ S1) \times 100$$

Table 1

| | ZrO₂ phase (mole%) | | | Al₂O₃ phase (vol%) | Average Grain Size (μm) | |
| | CeO₂ | TiO₂ | CaO | | ZrO₂ | Al₂O₃ |
|---|---|---|---|---|---|---|
| Comparative Example 1 | 11 | 0.05 | 0.16 | 0 | 2.50 | ---- |
| Comparative Example 2 | 11 | 0.05 | 0.16 | 10 | 1.35 | 0.23 |
| Example 1 | 11 | 0.05 | 0.16 | 20 | 0.43 | 0.24 |
| Example 2 | 11 | 0.05 | 0.16 | 30 | 0.23 | 0.26 |
| Example 3 | 11 | 0.05 | 0.16 | 40 | 0.21 | 0.27 |
| Example 4 | 11 | 0.05 | 0.16 | 50 | 0.19 | 0.27 |
| Example 5 | 11 | 0.05 | 0.16 | 60 | 0.18 | 0.28 |
| Example 6 | 11 | 0.05 | 0.16 | 70 | 0.17 | 0.29 |
| Comparative Example 3 | 11 | 0.05 | 0.16 | 80 | 0.16 | 0.30 |

Table 2

| | Blending Strength (MPa) | Fracture Toughness (MPa·m$^{1/2}$) | Vickers Hardness (GPa) | Wear factor (mm³/Nm x 10⁻⁷) | First Dispersion Ratio (%) | Second Dispersion Ratio (%) | Third Dispersion Ratio (%) |
|---|---|---|---|---|---|---|---|
| Comparative Example 1 | 750 | 18.0 | 8.5 | 230 | --- | --- | --- |
| Comparative Examples 2 | 1080 | 17.2 | 9.7 | 48.2 | 3.5 | 6.5 | 1.4 |
| Example 1 | 1260 | 16.7 | 10.8 | 0.135 | 3.4 | 6.4 | 1.4 |
| Example 2 | 1380 | 15.8 | 12.0 | 0.048 | 3.3 | 6.3 | 1.3 |
| Example 3 | 1430 | 14.5 | 13.1 | 0.036 | 3.1 | 6.1 | 1.2 |
| Example 4 | 1410 | 13.2 | 14.3 | 0.028 | 2.8 | 5.9 | 1.1 |
| Example 5 | 1340 | 11.8 | 15.4 | 0.051 | 2.3 | 5.6 | 0.9 |
| Example 6 | 1220 | 10.2 | 16.6 | 0.074 | 1.7 | 5.1 | 0.7 |
| Comparative Example 3 | 900 | 7.9 | 17.7 | 2.65 | 0.9 | 4.5 | 0.4 |

In addition, with respect to each of Examples 1 to 6 and Comparative Examples 1 to 3, average grain sizes of ZrO₂ grains and Al₂O₃ grains of the sintered body were measured by the TEM/SEM observation. To evaluate mechanical properties of the composite ceramic material, test specimens having the dimensions of 4mm x 3mm x 40mm were prepared from the sintered body, and then 3-point bending strength and fracture toughness were measured at room temperature. The fracture toughness was measured by the IF method. Results are listed in Tables 1 and 2.

Moreover, to evaluate wear resistance of the composite ceramic material, a pin-on-disc test was performed in the presence of distilled water as a lubricant. The pin and the disc are made of the same composite ceramic material. The pin is provided with a cylinder solid having a diameter of 5 mm and a length of 15 mm, and a circular cone having an apical angle of 30° and formed on a top of the cylinder solid. The top end of the circular cone has a flat mirror area with a diameter of 1.5 mm, which is used as a sliding surface. A surface roughness of this sliding surface is smaller than 0.005 μm Ra.

On the other hand, the disc has a diameter of 50 mm and a thickness of 8 mm. A sliding surface of the disc to be made contact with the pin is a mirror polished surface having a surface roughness smaller than 0.005 μm Ra. After the pin was placed on a circumference having a radius of 22 mm from the disc center on the disc, the pin-on-disc test was performed at a disc rotational speed of 60 mm/ sec. A load applied to the pin is 60N, and a sliding distance is constant (25 km). Since the diameter of the top end of the pin is 1.5 mm, an initial friction pressure on the top end of the pin is 33MPa. The pin-on-disc test was repeated three times under the same condition. An average value of the tests was adopted as data.

A reduction in weight of the pin was measured after the test, and a wear factor (Wf) was calculated by the following equation.

$$\mathbf{Wf} = (\mathbf{W1} - \mathbf{W2}) / \mathbf{P} \cdot \mathbf{L} \cdot \rho$$

Where,

Wf : wear factor (mm3/Nm)
W1 : dry weight (g) of pin before test
W2 : dry weight (g) of pin after test
P : load (N)
L : sliding distance (m)
$\rho$ : density (g/mm3) of test specimen

In addition, Vickers hardness of the composite ceramic material was measured. Measurement results of the wear factor and the hardness are shown in Table 2.

As understood from results of Tables 1 and 2, the sintered bodies of Examples 1 to 6 containing 20 to 70 vol% of $Al_2O_3$ have the first dispersion ratio (W1) greater than 1.5%, second dispersion ration (W2) greater than 4%, and the third dispersion ratio (W3) greater than 0. 3%. In addition, these sintered bodies demonstrate excellent mechanical properties of a bending strength greater than 1200 MPa and a fracture toughness higher than 10.0 MPa $\cdot$ m$^{1/2}$.

On the other hand, since the sintered body of Comparative Example 1 does not contain the $Al_2O_3$ phase, it has excellent fracture toughness. However, the bending strength of the sintered body is considerably low. The sintered body of Comparative Example 2 has excellent fracture toughness and the first to third dispersion ratios substantially equal to the sintered body of Example 1. However, the average grain size (= 1.35 $\mu$m) of the $ZrO_2$ grains of Comparative Example 2 is much greater than the average grain size (= 0.43 $\mu$m) of the $ZrO_2$ grains of Example 1. This suggests that the grain growth of the $ZrO_2$ grains can not be sufficiently inhibited by using such a small amount of $Al_2O_3$. As a result, the sintered body of Comparative Example 2 has a relatively low mechanical strength, and a tendency of increasing variations in mechanical properties. Thus, it is difficult to provide the composite ceramic material that is excellent in both of strength and toughness. With respect to the sintered body of Comparative Example 3, since the excessive amount of $Al_2O_3$ was used, both of strength and toughness considerably lowered. In addition, the number of the $Al_2O_3$ grains dispersed in the $ZrO_2$ grains, i.e., the first dispersion ratio (W1) is extremely low. This suggests that the composite particles can not be efficiently dispersed in the composite ceramic material in the case of using such a large amount of $Al_2O_3$.

As described above, the concern of the present invention is to provide the ceramic material having excellent wear resistance and hardness, while maintaining high strength and toughness under the larger amount of $Al_2O_3$ than heretofore. The results of Table 2 show that both of wear resistance and hardness can be highly improved when the $Al_2O_3$ content is in the range of 20 to 70 vol%. On the contrary, the sintered body of Comparative Example 2 shows a deterioration in wear resistance due to the deficiency of $Al_2O_3$ and the increase in average grain size of the $ZrO_2$ grains. In addition, the sintered body of Comparative Example 3 shows poor mechanical strength and toughness as well as the deterioration in wear resistance due to the excessive amount of $Al_2O_3$ in the composite ceramic material.

(Examples 7 to 21)

A $ZrO_2$-$Al_2O_3$ composite ceramic materials of each of Examples 7 to 21 was produced by the following method. That is, as listed in table 3, a tetragonal $ZrO_2$ powder having a specific surface area of 15 m$^2$/g and containing a $CeO_2$ amount of 10 to 12 mol% or containing the $CeO_2$ amount of 10 to 12 mol%, $TiO_2$ amount of 0.02 to 1 mol%, and a CaO amount of 0.02 to 0.5 mol% was used as the first component for providing a $ZrO_2$ phase of the composite ceramic material. On the other hand, as a second component for providing an $Al_2O_3$ phase of the composite ceramic material, a composite powder prepared by the following process was used. That is, a part of the above-described tetragonal $ZrO_2$ powder was added to a hydrochloric acid solution of aluminum chloride ($AlCl_3$) such that a mixture ratio by volume of $Al_2O_3$: $ZrO_2$ is 70 : 30. Next, an aqueous solution of sodium hydroxide was added to a resultant mixed solution, and hydrolyzed to obtain a precipitate. The precipitate was washed with water, and then dried. Next, the dried precipitate was calcined at 1000 °C in the air for 2 hours. The thus obtained calcined powder was ball-milled in an ethanol solvent for 24 hours, and then dried to obtain the composite powder.

The remaining tetragonal $ZrO_2$ powder was mixed with the composite powder such that an $Al_2O_3$ amount in the composite ceramic material is 40 vol%. A resultant mixture was ball-milled in an ethanol solvent for 24 hours, and then dried to obtain a powder for sintering. The powder for sintering was molded into a required shape by uniaxial pressing, and then sintered by pressureless sintering to obtain a sintered body, as in the case of Example 1.

With respect to each of Examples 7 to 21, the sintered body has a relative density of more than 99%. By X-ray diffraction analysis, it was confirmed that the $ZrO_2$ phase of the respective sintered body is composed of 90 vol% or more of tetragonal $ZrO_2$ and the balance of monoclinic $ZrO_2$. In addition, by SEM (scanning electron microscope) and TEM (transmission electron microscope) observations, it was confirmed that the sintered body of each of Examples 7 to 21 comprises composite particles dispersed therein, each of which has a triple nanocomposite structure that an $Al_2O_3$ grain containing a fine $ZrO_2$ grain therein is trapped within a $ZrO_2$ grain. As in the case of Example 1, the first to third dispersion ratios were determined with respect to each of Examples 7 to 21. Results are shown in Table 4.

Table 3

|  | $ZrO_2$ phase (mol%) | | | $Al_2O_3$ phase (vol%) | Average Grain Size (μm) | |
| --- | --- | --- | --- | --- | --- | --- |
|  | $CeO_2$ | $TiO_2$ | CaO | | $ZrO_2$ | $Al_2O_3$ |
| Example 7 | 10 | 0.00 | 0.00 | 40 | 0.21 | 0.20 |
| Example 8 | 10 | 0.05 | 0.03 | 40 | 0.24 | 0.22 |
| Example 9 | 10 | 0.30 | 0.15 | 40 | 0.33 | 0.25 |
| Example 10 | 10 | 0.70 | 0.35 | 40 | 0.45 | 0.28 |
| Example 11 | 10 | 1.00 | 0.50 | 40 | 0.50 | 0.30 |
| Example 12 | 11 | 0 | 0 | 40 | 0.21 | 0.20 |
| Example 13 | 11 | 0.03 | 0.02 | 40 | 0.23 | 0.21 |
| Example 14 | 11 | 0.20 | 0.10 | 40 | 0.29 | 0.24 |
| Example 15 | 11 | 0.60 | 0.30 | 40 | 0.43 | 0.28 |
| Example 16 | 11 | 0.90 | 0.45 | 40 | 0.48 | 0.29 |
| Example 17 | 12 | 0 | 0 | 40 | 0.21 | 0.20 |
| Example 18 | 12 | 0.02 | 0.01 | 40 | 0.22 | 0.21 |
| Example 19 | 12 | 0.10 | 0.05 | 40 | 0.26 | 0.23 |
| Example 20 | 12 | 0.50 | 0.25 | 40 | 0.40 | 0.27 |
| Example 21 | 12 | 0.80 | 0.40 | 40 | 0.47 | 0.29 |

Table 4

|  | Bending Strength (MPa) | Fracture Toughness (MPa·m$^{1/2}$) | First Dispersion Ratio (%) | Second Dispersion Ratio (%) | Third Dispersion Ratio (%) |
| --- | --- | --- | --- | --- | --- |
| Example 7 | 1100 | 18.0 | 2.3 | 4.8 | 1.3 |
| Example 8 | 1350 | 17.6 | 2.8 | 5.9 | 1.6 |
| Example 9 | 1260 | 17.4 | 3.0 | 6.4 | 1.7 |
| Example 10 | 1240 | 17.2 | 3.1 | 6.6 | 1.8 |
| Example 11 | 1210 | 17.1 | 3.3 | 7.0 | 1.9 |
| Example 12 | 1220 | 14.8 | 2.3 | 5.0 | 1.3 |
| Example 13 | 1420 | 14.5 | 2.8 | 5.9 | 1.6 |
| Example 14 | 1410 | 14.3 | 2.9 | 6.1 | 1.6 |

(continued)

|  | Bending Strength (MPa) | Fracture Toughness (MPa·m$^{1/2}$) | First Dispersion Ratio (%) | Second Dispersion Ratio (%) | Third Dispersion Ratio (%) |
|---|---|---|---|---|---|
| Example 15 | 1310 | 14.1 | 3.0 | 6.4 | 1.7 |
| Example 16 | 1290 | 14.0 | 3.2 | 6.8 | 1.8 |
| Example 17 | 1410 | 11.0 | 2.3 | 4.8 | 1.3 |
| Example 18 | 1570 | 10.7 | 2.8 | 5.9 | 1.6 |
| Example 19 | 1540 | 10.6 | 2.9 | 6.1 | 1.7 |
| Example 20 | 1380 | 10.5 | 3.0 | 6.4 | 1.7 |
| Example 21 | 1360 | 10.4 | 3.2 | 6.8 | 1.8 |

In addition, with respect to each of Examples 7 to 21, average grain sizes of the $ZrO_2$ grains and the $Al_2O_3$ grains of the sintered body were measured by the SEM/TEM observation. The average grain size of the $ZrO_2$ grains is in a range of 0.2 to 0.5 $\mu$m, and the average grain size of the $Al_2O_3$ grains is 0.3 $\mu$m or less. To evaluate mechanical properties of the composite ceramic material, test specimens having dimensions of 4mm x 3mm x 40mm were prepared from the sintered body, and 3-point bending strength and fracture toughness were measured at room temperature. The fracture toughness was measured by the IF method. Results are shown in Tables 3 and 4.

The results of Tables 3 and 4 suggest that the bending strength can be further improved by using slight amounts of $TiO_2$ and CaO in addition to $CeO_2$ as the stabilizer, without deteriorating the fracture toughness.

(Examples 22 to 27)

A $ZrO_2$-$Al_2O_3$ composite ceramic materials of each of Examples 22 to 27 was produced by the following method. That is, as the first component for providing a $ZrO_2$ phase of the composite ceramic material, a tetragonal $ZrO_2$ powder having a specific surface of 15 m$^2$/g and containing 11 mol% of $CeO_2$, 0.05 mol% of $TiO_2$, and 0.13 mol% of CaO was used. On the other hand, as a second component for providing an $Al_2O_3$ phase of the composite ceramic material, a composite powder comprised of a θ-$Al_2O_3$ powder having a specific surface of 100 m$^2$/g and a part of the above-described tetragonal $ZrO_2$ powder was used. A mixture ratio by volume of the θ-$Al_2O_3$ powder and the tetragonal $ZrO_2$ powder was changed in a range of 95 : 5 to 50 : 50, as shown in Table 5.

The composite powder was prepared by the following procedures. That is, required amounts of the θ-$Al_2O_3$ powder and the above-described tetragonal $ZrO_2$ powder were ball-milled in an ethanol solvent for 24 hours, and then dried to obtain a mixed powder. Subsequently, the mixed powder was calcined at 1000 °C in the air for 2 hours. The thus obtained calcined powder was further ball-milled in an ethanol solvent for 24 hours, and then dried to obtain the composite powder.

The remaining tetragonal $ZrO_2$ powder was mixed with the composite powder such that an $Al_2O_3$ content in the composite ceramic material is 50 vol%. A resultant mixture was ball-milled in an ethanol solvent for 24 hours, and the dried to obtain a powder for sintering. The powder for sintering was molded into a required shape by uniaxial pressing, and then sintered by pressureless sintering to obtain a sintered body, as in the case of Example 1.

With respect to each of Examples 22 to 27, the sintered body has a relative density of more than 99%. By X-ray diffraction analysis, it was confirmed that the $ZrO_2$ phase of the respective sintered body is composed of 90 vol% or more of tetragonal $ZrO_2$ and the balance of monoclinic $ZrO_2$. In addition, by SEM (scanning electron microscope) and TEM (transmission electron microscope) observations, it was confirmed that the sintered body of each of Examples 22 to 27 comprises composite particles dispersed therein, each of which has a triple nanocomposite structure that an $Al_2O_3$ grain containing a fine $ZrO_2$ grain therein is trapped within a $ZrO_2$ grain. As in the case of Example 1, the first to third dispersion ratios were determined with respect to each of Examples 22 to 27. Results are shown in Table 6.

In addition, with respect to each of Examples 22 to 27, average grain sizes of $ZrO_2$ grains and $Al_2O_3$ grains of the sintered body were measured by the SEM / TEM observation. The average grain size of the $ZrO_2$ grains is in a range of 0.2 to 0.3 $\mu$m, and the average grain size of the $Al_2O_3$ grains is 0.3 $\mu$m or less. To evaluate mechanical properties of the composite ceramic material, test specimens having dimensions of 4mm x 3mm x 40mm were prepared from the sintered body, and 3-point bending strength and fracture toughness were measured at room temperature. The fracture toughness was measured by the IF method. Results are shown in Tables 5 and 6.

The results of Tables 5 and 6 suggest that when the mixture ratio of $Al_2O_3$ and tetragonal $ZrO_2$ in the composite powder is in the range of 95 : 5 to 50 : 50, and particularly 90 : 10 to 60 : 40, the $ZrO_2$ grains can be efficiently trapped

within the $Al_2O_3$ grains. In addition, the number of the $Al_2O_3$ grains each containing the $ZrO_2$ grain therein, i.e., the second dispersion ratio (W2) can be increased by use of the composite powder with the above mixture ratio. Furthermore, by selecting a suitable mixture ratio of $Al_2O_3$ and tetragonal $ZrO_2$ in the composite powder, it is possible to obtain the composite ceramic material having a further improved strength, while keeping the toughness constant.

As understood from the above Examples, the $ZrO_2$-$Al_2O_3$ composite ceramic material of the present invention is characterized by comprising composite particles dispersed therein, each of which has a triple nanocomposite structure that an $Al_2O_3$ grain containing a fine $ZrO_2$ grain therein is trapped within a larger $ZrO_2$ grain. The formation of this nanocomposite structure provides further improvements in wear resistance, hardness, strength and toughness of the $ZrO_2$-$Al_2O_3$ ceramic material under a larger amount of $Al_2O_3$ than heretofore. Therefore, the composite ceramic material of the present invention is expected to be preferably utilized in various application fields, for example, parts for industrial machine such as ferrule for optical fiber connector, bearings and dies, cutting tools such as scissor and saw blade, stationery goods, chemical goods such as mechanical seal and milling media, goods for sport, medical equipments such as surgical knife, biomaterial parts such as artificial joint, artificial bone, artificial dental root, abutment and crown.

Table 5

| | $ZrO_2$ phase (mol%) | | | $Al_2O_3$ phase vol% ($Al_2O_3$ : $ZrO_2$) | Average Grain Size ($\mu$m) | |
| --- | --- | --- | --- | --- | --- | --- |
| | $CeO_2$ | $TiO_2$ | CaO | | $ZrO_2$ | $Al_2O_3$ |
| Example 22 | 11 | 0.05 | 0.13 | 50 (95 : 5) | 0.25 | 0.30 |
| Example 23 | 11 | 0.05 | 0.13 | 50 (90 : 10) | 0.23 | 0.28 |
| Example 24 | 11 | 0.05 | 0.13 | 50 (80 20) | 0.22 | 0.27 |
| Example 25 | 11 | 0.05 | 0.13 | 50 (70 : 30) | 0.21 | 0.26 |
| Example 26 | 11 | 0.05 | 0.13 | 50 (60 : 40) | 0.22 | 0.27 |
| Example 27 | 11 | 0.05 | 0.13 | 50 (50 : 50) | 0.23 | 0.28 |

Table 6

| | Bending Strength (MPa) | Fracture Toughness (MPa·m$^{1/2}$) | First Dispersion Ratio (%) | Second Dispersion Ratio (%) | Third Dispersion Ratio (%) |
| --- | --- | --- | --- | --- | --- |
| Example 22 | 1230 | 13.0 | 2.9 | 4.2 | 0.3 |
| Example 23 | 1340 | 13.1 | 3.0 | 5.1 | 0.6 |
| Example 24 | 1430 | 13.3 | 3.1 | 6.1 | 1.2 |
| Example 25 | 1430 | 13.3 | 3.2 | 6.2 | 1.8 |
| Example 26 | 1380 | 13.2 | 3.2 | 5.8 | 2.4 |
| Example 27 | 1260 | 12.9 | 3.1 | 4.9 | 2.8 |

## Claims

1. A $ZrO_2$-$Al_2O_3$ composite ceramic material comprising a $ZrO_2$ phase composed of 90 vol% or more of tetragonal $ZrO_2$, and an $Al_2O_3$ phase, wherein an amount of said $Al_2O_3$ phase in the composite ceramic material is in a range of 20 to 70 vol%, and the composite ceramic material comprises composite grains dispersed therein, each of which has a structure that an $Al_2O_3$ grain containing a fine $ZrO_2$ grain therein is trapped within a $ZrO_2$ grain, wherein a ratio of the number of the $Al_2O_3$ grains each existing in said composite particle and having the fine $ZrO_2$ grain therein relative to the number of the entire $Al_2O_3$ grains dispersed in the composite ceramic material is 0,3 % or more.

2. The composite ceramic material as set forth in claim 1, wherein said $ZrO_2$ phase contains 10 to 12 mol% of $CeO_2$ as a stabilizer.

3. The composite ceramic material as set forth in claim 1 or 2, wherein a first dispersion ratio of the number of AlzOs grains dispersed in $ZrO_2$ grains relative to the number of the entire $Al_2O_3$ grains dispersed in the composite ceramic material is 1,5% or more.

4. The composite ceramic material as set forth in any one of claims 1 to 3, wherein a second dispersion ratio of the number of $ZrO_2$ grains dispersed in $Al_2O_3$ grains relative to the number of the entire $ZrO_2$ grains dispersed in the composite ceramic material is 4% or more.

5. The composite ceramic material as set forth in any one of claims 1 to 4, wherein an average grain size of said $ZrO_2$ phase is in a range of 0.1 to 1 $\mu$m, and an average grain size of said $Al_2O_3$ phase is in a range of 0,1 to 0,5 $\mu$m.

6. A method of producing a $ZrO_2$-$Al_2O_3$ composite ceramic material comprising a $ZrO_2$ phase composed of 90 vol% or more of tetragonal $ZrO_2$, and an $Al_2O_3$ phase, the method comprising the steps of

   - mixing a first powder for providing said $ZrO_2$ phase with a second powder for providing said $Al_2O_3$ phase such that an amount of said $Al_2O_3$ phase in the composite ceramic material is in a range of 20 to 70 vol%the second powder containing $Al_2O_3$ particles each having a fine $ZrO_2$ particle therein;
   - molding a resultant mixture in a desired shape to obtain a green compact; and
   - sintering said green compact in an oxygen-containing atmosphere, so that the composite ceramic material comprises composite particles dispersed therein, each of which has a structure that an $Al_2O_3$ grain containing a fine $ZrO_2$ gain therein is trapped within a $ZrO_2$ grain, and a ratio of the number of the $Al_2O_3$ grains existing in said composite particle and having the fine $ZrO_2$ grain therein relative to the number of the entire $Al_2O_3$ grains dispersed in the composite ceramic material is 0,3% or more.

7. The method as set forth in claim 6, wherein the first powder comprises a $ZrO_2$ powder containing 10 to 12 mol% of $CeO_2$ as a s stabilizer.

8. The method as set forth in any one of the claims 6 or 7, wherein a preparation process of the second powder comprises a step of adding a $ZrO_2$ powder to at least one selected from a $\theta$-$Al_2O_3$ powder and a $\gamma$-$Al_2O_3$ powder having a specific surface area of 50 to 400 $m^2$/g to obtain a mixed powder, and then calcining the mixed powder in an oxygen atmosphere at a temperature of 800°C to 1300°C.

9. The method as set forth in any of the claims 6 or 7, wherein a preparation process of the second powder comprises the steps of adding a $ZrO_2$ powder to one of an aqueous solution of an aluminum salt and an organic solution of an aluminum alkoxide, hydrolyzing a resultant mixture to obtain a precipitate, and drying the precipitate, and then calcining the precipitate in an oxygen containing atmosphere at a temperature of from 800°C to 1300°C.

10. The method as set forth in any one of claims 6 or 7, wherein a preparation process of the second powder comprises the steps of adding an aqueous solution of a zirconium salt to one of an aqueous solution of an aluminum salt and an organic solution of an aluminum alkoxide, hydrolyzing a resultant mixture to obtain a precipitate, and drying the precipitate, and then calcining the precipitate in an oxygen containing atmosphere at a temperature of from 800°C to 1300°C.

11. The method as set forth in any one of claims 6 to 10, wherein the second powder is mainly composed of $\alpha$-$Al_2O_3$ particles having an average particle size of 0,3 $\mu$m or less, each of which contains a fine $ZrO_2$ particle therein.

12. The method as set forth in any one of claims 6 to 11, wherein a volume ratio of $Al_2O_3$ : $ZrO_2$ in the second powder is in a range of 95 : 5 to 50 : 50.

**Patentansprüche**

1. $ZrO_2$-$Al_2O_3$ keramisches Verbundmaterial mit einer $ZrO_2$-Phase, die zu 90 Vol.-% oder mehr von tetragonalem $ZrO_2$ gebildet ist, und eine $Al_2O_3$-Phase umfasst, wobei eine Menge der $Al_2O_3$-Phase in dem keramischen Verbundmaterial in einem Bereich von 20-70 Vol.% ist und das keramische Verbundmaterial darin verteilte Verbundkörner umfasst, wobei jedes davon eine Struktur aufweist, bei der ein $Al_2O_3$-Korn eine feinkörniges $ZrO_2$-Korn darin aufweist und in einem $ZrO_2$-Korn eingeschlossen ist, wobei ein Verhältnis der Anzahl der $Al_2O_3$-Körner, die in dem Verbundteilchen vorkommen und das feinkörnige $ZrO_2$-Korn darin aufweisen, relativ zu der Anzahl aller $Al_2O_3$-Kör-

**EP 1 580 178 B1**

ner, die in dem keramischen Verbundmaterial aufgelöst sind, 0,3 % oder mehr beträgt.

2. Keramisches Verbundmaterial nach Anspruch 1, wobei die $ZrO_2$-Phase 10-12 mol.-% an $CeO_2$ als einen Stabilisator umfasst.

3. Keramisches Verbundmaterial nach einem der Ansprüche 1 oder 2, wobei ein erstes Dispersionsverhältnis der Anzahl der AlzOs-Körner, die in $ZrO_2$-Körnern dispergiert sind, relativ zu der Anzahl aller $Al_2O_3$-Körner, die in dem keramischen Verbundmaterial verteilt sind, 1, 5 % oder mehr beträgt.

4. Keramisches Verbundmaterial nach einem der Ansprüche 1-3, wobei ein zweites Dispersionsverhältnis der Anzahl von $ZrO_2$-Körnern, die in $Al_2O_3$-Körnern verteilt sind, relativ zu der Anzahl aller $ZrO_2$-Körner, die in dem keramischen Verbundmaterial verteil sind, 4 % oder mehr beträgt.

5. Keramisches Verbundmaterial nach einem der Ansprüche 1-4, wobei eine durchschnittliche Korngröße der $ZrO_2$-Phase in einem Bereich von 0,1 bis 1 $\mu$m liegt und eine durchschnittliche Korngröße der $Al_2O_3$-Phase in einem Bericht von 0,1-0,5 $\mu$m liegt.

6. Verfahren zum Herstellen eines $ZrO_2$-$Al_2O_3$ keramischen Verbundmaterials mit einer $ZrO_2$-Phase, die zu 90 Vol.-% oder mehr von tetragonalen $ZrO_2$ gebildet ist, und eine $Al_2O_3$-Phase umfasst, wobei das Verfahren folgende Schritte umfasst:

Vermischen eines ersten Pulvers zum Bereitstellen der $ZrO_2$-Phase mit einem zweiten Pulver zum Bereitstellen der $Al_2O_3$-Phase, so dass eine Menge der $Al_2O_3$-Phase in dem keramischen Verbundmaterial in einem Bereich von 20 bis 70 Vol.-% liegt, wobei das zweite Pulver $Al_2O_3$-Teilchen umfasst, von denen jedes ein feinkörniges $ZrO_2$-Teilchen darin umfasst;
Formen einer resultierenden Mischung in eine gewünschte Form, um einen Grünkörper zu erhalten; und
Sintern des Grünkörpers in einer Sauerstoff enthaltenden Atmosphäre, so dass das keramische Verbundmaterial darin verteilte Verbundteilchen aufweist, wobei jedes eine Struktur aufweist, bei der ein $Al_2O_3$-Korn ein feinkörniges $ZrO_2$-Korn enthält und in einem $ZrO_2$-Korn eingeschlossen ist, und wobei ein Verhältnis der Anzahl von $Al_2O_3$-Körnern, die in dem Verbundteilchen vorkommen und ein feinkörniges $ZrO_2$-Korn darin enthalten, relativ zu der Anzahl der gesamten $Al_2O_3$-Körnern, die in dem keramischen Verbundmaterial dispergiert sind, 0,3 % oder mehr beträgt.

7. Verfahren nach Anspruch 6, wobei das erste Pulver ein $ZrO_2$-Pulver umfasst, welches 10-12 mol-% von $CeO_2$ als Stabilisator enthält.

8. Verfahren nach einem der Ansprüche 6 oder 7, wobei ein Vorbereitungsprozess für das zweite Pulver einen Schritt des Hinzufügens eines $ZrO_2$-Pulvers zu einem $\theta$-$Al_2O_3$-Pulver oder einem $\gamma$-$Al_2O_3$-Pulver mit einer spezifischen Oberfläche von 50 bis 400 $m^2$/g umfasst, um ein Mischpulver zu erhalten, wobei daran anschließend das Mischpulver in einer Sauerstoffatmosphäre bei einer Temperatur von 800°C bis 1300°C kalziniert wird.

9. Verfahren nach einem der Ansprüche 6 oder 7, wobei ein Vorbereitungsprozess für das zweite Pulver folgende Schritte umfasst: Hinzufügen eines $ZrO_2$-Pulvers zu einem aus dem Folgenden: einer wässrigen Lösung von Aluminiumsalz und einer organischen Lösung von Aluminiumalkoxid, Hydrolysieren einer resultierenden Mischung, um eine Ausfällung zu erhalten, und Trocknen des Ausfallproduktes und daran anschließend Kalzinieren der Ausfällung in einer Sauerstoff enthaltenden Atmosphäre bei einer Temperatur von 800°C bis 1300°C.

10. Verfahren nach einem der Ansprüche 6 oder 7, wobei der Vorbereitungsprozess für das zweite Pulver die folgenden Schritte umfasst: Hinzufügen einer wässrigen Lösung von Zirkoniumsalz zu einer wässrigen Lösung von Aluminiumsalz oder zu einer organischen Lösung von Aluminiumalkoxid, Hydrolysieren einer resultierenden Mischung, um eine Ausfällung zu erhalten, und Trocknen der Ausfällung und daran anschließend Kalzinieren der Ausfällung in einer Sauerstoff enthaltenden Atmosphäre bei einer Temperatur von 800°C bis 1300°C.

11. Verfahren nach einem der Ansprüche 6-10, wobei das zweite Pulver im Wesentlichen aus $\alpha$-$Al_2O_3$-Teilchen zusammengesetzt ist, die eine durchschnittliche Teilchengröße von 0,3 $\mu$m oder weniger aufweisen, wobei jedes davon ein feinkörniges $ZrO_2$-Teilchen darin enthält.

12. Verfahren nach einem der Ansprüche 6-11, wobei ein Volumenverhältnis von $Al_2O_3$: $ZrO_2$ in dem zweiten Pulver

**15**

in einem Bereich von 95:5 bis 50:50 liegt.

**Revendications**

1. Matériau céramique composite $ZrO_2$-$Al_2O_3$ comprenant une phase de $ZrO_2$ composée à 90 % en volume ou plus de $ZrO_2$ tétragonal, une phase de $Al_2O_3$, où une quantité de ladite phase de $Al_2O_3$ dans le matériau céramique composite est dans une plage de 20 à 70 % en volume, et le matériau céramique composite comprend des grains de composite dispersés en son intérieur, dont chacun a une structure telle qu'un grain de $Al_2O_3$ contenant un grain fin de $ZrO_2$ en son intérieur est piégé au sein d'un grain de $ZrO_2$, où un rapport entre le nombre des grains de $Al_2O_3$ existant chacun dans ladite particule composite et comportant le grain fin de $ZrO_2$ en son intérieur et le nombre total des grains de $Al_2O_3$ dispersés dans le matériau céramique composite est de 0,3 % ou plus.

2. Matériau céramique composite selon la revendication 1, dans lequel ladite phase de $ZrO_2$ contient 10 à 12 % en mol de $CeO_2$ en tant que stabilisant.

3. Matériau céramique composite selon la revendication 1 ou 2, dans lequel un premier rapport de dispersion entre le nombre de grains de $Al_2O_3$ dispersés dans les grains de $ZrO_2$ et le nombre total des grains de $Al_2O_3$ dispersés dans le matériau céramique composite est de 1,5 % ou plus.

4. Matériau céramique composite selon l'une quelconque des revendications 1 à 3, dans lequel un second rapport de dispersion du nombre de grains de $ZrO_2$ dispersés dans les grains de $Al_2O_3$ et le nombre total des grains de $ZrO_2$ dispersés dans le matériau céramique composite est de 4 % ou plus.

5. Matériau céramique composite selon l'une quelconque des revendications 1 à 4, dans lequel une taille moyenne de grain de ladite phase de $ZrO_2$ est dans une plage de 0,1 à 1 $\mu$m, et une taille moyenne de grain de ladite phase de $Al_2O_3$ est dans une plage de 0,1 à 0,5 $\mu$m.

6. Procédé de production d'un matériau céramique composite $ZrO_2$-$Al_2O_3$ comprenant une phase de $ZrO_2$ composée à 90 % en volume ou plus de $ZrO_2$ tétragonal, et une phase de $Al_2O_3$, le procédé comprenant les étapes consistant à :

   - mélanger une première poudre pour fournir ladite phase de $ZrO_2$ avec une seconde poudre pour fournir ladite phase de $Al_2O_3$ de telle sorte qu'une quantité de ladite phase de $Al_2O_3$ dans le matériau céramique composite est dans une plage de 20 à 70 % en volume, la seconde poudre contenant des particules de $Al_2O_3$ ayant chacune une particule fine de $ZrO_2$ en son intérieur ;
   - mouler un mélange résultant dans la forme souhaitée pour obtenir un compact vert ; et
   - fritter ledit compact vert dans une atmosphère contenant de l'oxygène, de sorte que le matériau céramique composite comprend des particules composites dispersées en son intérieur, dont chacune a une structure telle qu'un grain de $Al_2O_3$ contenant un grain fin de $ZrO_2$ en son intérieur est piégé au sein d'un grain de $ZrO_2$, et un rapport entre le nombre des grains de $Al_2O_3$ existant dans ladite particule composite et comportant le grain fin de $ZrO_2$ en son intérieur et le nombre total des grains de $Al_2O_3$ dispersés dans le matériau céramique composite est de 0,3 % ou plus.

7. Procédé selon la revendication 6, dans lequel la première poudre comprend une poudre de $ZrO_2$ contenant 10 à 12 % en mol de $CeO_2$ en tant que stabilisant.

8. Procédé selon l'une quelconque des revendications 6 ou 7, dans lequel un processus de préparation de la seconde poudre comprend une étape consistant à ajouter une poudre de $ZrO_2$ à au moins un élément choisi parmi une poudre de $\delta$-$Al_2O_3$ et une poudre de $\gamma$-$Al_2O_3$ ayant une surface spécifique de 50 à 400 $m^2$/g pour obtenir une poudre mixte, puis calciner la poudre mixte dans une atmosphère d'oxygène à une température de 800 °C à 1 300 °C.

9. Procédé selon l'une quelconque des revendications 6 ou 7, dans lequel un processus de préparation de la seconde poudre comprend les étapes consistant à ajouter une poudre de $ZrO_2$ à un élément parmi une solution aqueuse d'un sel d'aluminium et une solution organique d'un alcoxyde d'aluminium, hydrolyser un mélange résultant pour obtenir un précipité, et sécher le précipité, puis calciner le précipité dans une atmosphère contenant de l'oxygène à une température de 800 °C à 1 300 °C.

10. Procédé selon l'une quelconque des revendications 6 ou 7, dans lequel un processus de préparation de la seconde poudre comprend les étapes consistant à ajouter une solution aqueuse d'un sel de zirconium à un élément parmi une solution aqueuse d'un sel d'aluminium et une solution organique d'un alcoxyde d'aluminium, hydrolyser un mélange résultant pour obtenir un précipité, et sécher le précipité, puis calciner le précipité dans une atmosphère contenant de l'oxygène à une température de 800 °C à 1 300 °C.

11. Procédé selon l'une quelconque des revendications 6 à 10, dans lequel la seconde poudre est principalement composée de particules d'$\alpha$-Al$_2$O$_3$ ayant une taille moyenne de particule de 0,3 $\mu$m ou moins, dont chacune contient une particule fine de ZrO$_2$ en son intérieur.

12. Procédé selon l'une quelconque des revendications 6 à 11, dans lequel un rapport en volume Al$_2$O$_3$ : ZrO$_2$ dans la seconde poudre est dans une plage de 95 : 5 à 50 : 50.

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5246760 A
- US 5728636 A
- EP 1382586 A2
- US 5863850 A
- WO 0211780 A

**Non-patent literature cited in the description**

- **Nawa M. et al.** The Effect of TiO2 Addition on Strengthening and Toughening in Intragranular Type of 12Ce-TZP/Al2O3 Nanocomposites. *Journal of the European Ceramic Society,* 1998, vol. 18 (3), 209-219